# DEMANDE DE BREVET EUROPEEN

(11) **EP 4 119 135 A1**
(43) Date de publication de la demande: **18.01.2023**
(21) Numéro de dépôt: 21305978.5
(22) Date de dépôt: 13.07.2021
(51) Int. Cl.: A61K 31/07, A61P 1/14, A23L 29/206, A23L 29/219, A23L 29/244, A23L 33/00, A61K 36/185, A61K 45/06, A61K 31/736

(54) **METHODES POUR REEQUILIBRER LA FLORE INTESTINALE CHEZ LES PATIENTS SOUFFRANT DE LA MALADIE DE PARKINSON**

(71) Demandeur: Luxia Scientific, 77590 Bois le Roi (FR)
(72) Inventeur: PLASSAIS, Jonathan, 92600 ASNIERES (FR); CERVINO, Alessandra, 77590 BOIS LE ROI (FR)
(74) Mandataire: Regimbeau

(57) **Abrégé**

Les présents inventeurs ont mis en évidence qu'il existe une corrélation entre l'abondance relative de certains genres bactériens, déterminés à partir de prélèvements fécaux (analyse par séquençage 16S), et le contenu des prises alimentaires des individus prélevés. Ces informations leur ont permis d'établir une relation entre l'ingurgitation de certains micro-nutriments, en fonction de leur quantité journalière absorbée, et l'abondance relative de 5 genres bactériens d'intérêt, à savoir *Robesuria, Faecalibacterium, Blautia, Butyricicoccus,* et *Clostridium XVIII.* Ils proposent donc un procédé novateur pour augmenter l'abondance relative de ces genres bactériens au sein du microbiote intestinal d'un individu, caractérisé en ce qu'il comprend l'administration d'un complément alimentaire contenant des micro-nutriments particuliers, notamment, du bêta-carotène, du galactomannane, des omega-3, du glucomannane et/ou du butyrate. Ces micro-nutriments sont de préférence destinés à améliorer le bien-être digestif et l'état de santé global de patients souffrant de la maladie de Parkinson.

## Description

Les présents inventeurs ont mis en évidence qu'il existe une corrélation entre l'abondance relative de certains genres bactériens, déterminés à partir de prélèvements fécaux (analyse par séquençage 16S), et le contenu des prises alimentaires des individus prélevés. Ces informations leur ont permis d'établir une relation entre l'ingurgitation de certains micro-nutriments, en fonction de leur quantité journalière absorbée, et l'abondance relative de 5 genres bactériens d'intérêt, à savoir *Robesuria, Faecalibacterium, Blautia, Butyricicoccus,* et *Clostridium XVIII.* Ils proposent donc un procédé novateur pour augmenter l'abondance relative de ces genres bactériens au sein du microbiote intestinal d'un individu, caractérisé en ce qu'il comprend l'administration d'un complément alimentaire contenant des micro-nutriments particuliers, notamment, du bêta-carotène, du galactomannane, des omega-3, du glucomannane et/ou du butyrate. Ces micro-nutriments sont de préférence destinés à améliorer le bien-être digestif et l'état de santé global de patients souffrant de la maladie de Parkinson.

### DESCRIPTION DE L'ETAT DE LA TECHNIQUE

La maladie de Parkinson est une maladie neurodégénérative progressive, caractérisée principalement par la dégénérescence des neurones à dopamine situés dans la substance noire du cerveau ainsi que d'une pathologie de Lewy, une agrégation anormale de protéines α-synucléine sous forme de corps de Lewy ou de neurites de Lewy dans le système nerveux central (Dauer and Przedborski, 2003 [9]; Kalia and Lang, 2015 [12]; Raza et al., 2019 [19]). Des agrégats d'a-synucléine ont également été identifiés en dehors du système nerveux central, comme par exemple dans le plexus sous-muqueux à l'étage oesophagien, gastrique ou colique (Kupsky et al., 1987 [14]; Braak et al., 2006 [4]). Les neurones à dopamine sont spécifiques du contrôle des mouvements et de leur destruction résulte des symptômes moteurs comme la lenteur des mouvements, la rigidité ou encore des tremblements au repos (Kalia and Lang, 2015 [12]). A ces symptômes moteurs s'ajoutent régulièrement des symptômes non-moteurs, notamment des troubles neurospychiatriques (dépression, atteinte cognitive), des troubles du sommeil, dysautonomiques et gastro-intestinaux (Chaudhuri et al., 2006 [5]; Pfeiffer, 2016 [17]). La constipation est très fréquente chez les patients Parkinson. On estime sa prévalence de l'ordre de 50% à 60% (Moisan et al., 2018 [21]) et ce signe non-moteur est un des signes précurseurs de la maladie. Les recherches sur l'étiologie et la pathogénèse de la maladie de Parkinson se poursuivent, et certains chercheurs faisant même l'hypothèse que la maladie pourrait trouver son origine dans l'intestin, où des milliers de protéines sont produites par des bactéries et parmi elles les protéines « amyloid-like » maintenant connues pour augmenter les protéines α-synucléines (Friedland, 2015 [10]; Cryan et al., 2020 [8]).

Le principal traitement dans la maladie de Parkinson est un précurseur de dopamine, appelé lévodopa ou L-dopa. Cette molécule est administrée par voie digestive et atteindra le tissu cérébral où elle sera transformée par réaction enzymatique en dopamine grâce à la dopadécarboxylase. La disponibilité, dans le cerveau, de la dopamine obtenue par cette molécule est souvent perturbée par la présence de dopadécarboxylase périphérique. Ainsi la lévodopa est souvent coadministrée avec des inhibiteurs de cette enzyme comme la carbidopa ou le bensérazide, permettant à la fois d'augmenter la biodisponibilité de la lévodopa dans le cerveau, mais également d'ajuster la dose administrée et de réduire les effets secondaires. Un autre inhibiteur spécifique et réversible de la catéchol-O-méthyltransférase (COMT), l'entacapone, peut être également co-administrée avec la lévodopa, bloquant la transformation de la lévodopa en 3-O-méthyldopa. Si la lévodopa est principalement réservée aux patients souffrants de Parkinson depuis plusieurs années, d'autres antiparkinsoniens existent, par exemple les antagonistes à la dopamine (l'apomorphine ou la rotigotine par exemple). Les recherches se poursuivent afin d'identifier des molécules plus efficaces et surtout provoquant moins d'effets secondaires.

Il est maintenant reconnu que le système nerveux central, le système nerveux entérique et le microbiote dialoguent ensemble, notamment via le nerf vague. C'est ce qu'on appelle dorénavant l'axe « intestin-cerveau ». La description de cet axe repose principalement sur une des révolutions scientifiques majeures de ces dernières années qui est la description relativement exhaustive de la composition bactérienne du microbiote intestinal, rendue possible grâce aux nouvelles technologies de séquençage de l'ADN. A ce jour, même si de nombreuses recherches ont permis des avancées significatives dans le domaine, nous disposons d'une description non-exhaustive du microbiote intestinal, notamment parce que l'on estime à 40% le nombre d'espèces non-annotées, que les protéines produites par les bactéries ainsi que leur fonction sont encore mal décrites, et que l'interaction hôte-microbiote est encore un domaine en pleine exploration.

De nombreuses études cliniques observationnelles ont étudié l'association entre la composition du microbiote intestinal et la maladie de Parkinson. Ces études se basent principalement sur des résultats de métagénomique ciblée de l'ARN 16S. Dans une revue récente de la littérature, Boertien et al. (Boertien et al., 2019 [2]) ont montré que les familles *Verrucomicrobiaceae* et *Prevotellaceae* sont toutes deux reportées plusieurs fois comme étant associées à la maladie de Parkinson, la première étant diminuée et la seconde augmentée (Boertien et al., 2019 [2]). Cependant, au niveau des genres bactériens, les résultats comparés entre les études sont moins clairs. Seul *Akkermansia,* le seul genre connu à ce jour de la famille *Verrucomicrobiaceae,* est clairement associé avec la maladie, alors qu'aucun genre de la famille *Prevotellaceae* ne semble répétable. Cette étude montre la difficulté à obtenir des résultats cohérents entre les études, principalement dû à la variabilité technique de l'analyse du métagénome (biais d'extraction, différentes machines de séquençage, différentes bases de données taxonomiques) mais peut-être aussi à la variabilité liée aux différentes populations étudiées. Pour outrepasser ces problèmes et comparer les études entre elles, la meilleure méthode statistique est la méta-analyse.

Une méta-analyse a donc été réalisée pour évaluer quel(s) genre(s) bactérien(s) présente(nt) une abondance relative particulièrement diminuée au sein du microbiote des personnes atteintes de la maladie de Parkinson. Cette analyse a identifié pour la première fois cinq genres bactériens associés négativement avec la maladie. Il s'agit de : *Faecalibacterium, Blautia, Roseburia, Butyricicoccus,* et *Clostridium-XVII.*

Il existe un besoin urgent de développer des outils pour permettre de rééquilibrer cette flore bactérienne et ainsi améliorer l'état de santé global de ces patients.

Aucune étude n'a cependant proposé à ce jour de complément alimentaire dédié capable d'augmenter précisément l'abondance relative de ces cinq bactéries.

La présente invention répond à ce besoin, en proposant un complément alimentaire spécifiquement conçu pour augmenter l'abondance de ces cinq bactéries d'intérêt et ainsi permettre d'améliorer le bien-être digestif et/ou l'état de santé général chez des individus souffrant de la maladie de Parkinson.

### DESCRIPTION DETAILLEE DE L'INVENTION

Les exemples ci-dessous décrivent :
- La méta-analyse réalisée par les inventeurs pour mesurer l'abondance relative des genres bactériens afin d'identifier les profils bactériens associés à la maladie de Parkinson au sein d'une population âgée, nord-américaine et européenne (exemple 1).

Cette méta-analyse a mis en évidence une signature de 6 genres bactériens parmi lesquels 5, à savoir *Blautia, Butyricicoccus, Clostridium XVIII, Faecalibacterium* et *Roseburia,* sont significativement diminués chez les patients souffrant de la maladie de Parkinson.
- L'étude réalisée par les inventeurs pour identifier les aliments/nutriments qui permettent d'améliorer l'abondance relative de ces cinq genres bactériens (exemple 2).

Pour déterminer quels nutriments contribuent à l'augmentation de ces bactéries, les inventeurs ont analysé la composition du microbiote de centaines d'individus au regard de leur consommation alimentaire au cours du dernier mois. Pour ce faire, ils ont analysé leurs prélèvements fécaux par séquençage 16S (générant des données bio-informatiques relativement à la composition bactérienne de leur microbiote) ainsi que des questionnaires les renseignant sur leur consommation alimentaire au cours du dernier mois. Les fréquences moyennes de consommation journalière des aliments par individu, leurs apports quotidiens en nutriments (macronutriments, minéraux, vitamines, etc...) de ces individus ont été déterminés et confrontés aux profils bactériens des individus de la cohorte.

Les tests statistiques suivants ont été réalisés :
- Corrélations non paramétriques de Spearman entre les apports en nutriments calculés et chacune des cinq bactéries d'intérêt,
- Corrélations non paramétriques de Spearman entre les fréquences de consommation des aliments décrits dans le questionnaire alimentaire et chacune des cinq bactéries d'intérêt,
- Régressions ordinales entre les fréquences de consommation des aliments du questionnaire alimentaire et chacune des cinq bactéries d'intérêt.

Toutes ces corrélations ont permis d'identifier que la consommation de bêta-carotène est statistiquement associée à une flore bactérienne riche en bactéries du genre *Blautia, Faecalibacterium* et *Clostridium XVIII.* Les inventeurs proposent donc d'utiliser ce micro-nutriment dans un complément alimentaire ou au sein d'une alimentation spécifiquement adaptée pour améliorer le bien-être d'individus ayant une perte bactérienne au niveau de ce genre bactérien d'intérêt, et notamment, chez les patients atteints de la maladie de Parkinson.

Des études antérieures ont également montré qu'il est possible d'augmenter la quantité de bactéries de genre bactérien *Roseburia* en administrant une quantité efficace de glucomannane ou de galactomannane (La Rosa et al, 2019 [31]). Ces études ne proposent cependant pas d'administrer ces nutriments à des patients souffrant de la maladie de Parkinson.

Bien que le bénéfice cardiovasculaire et le métabolisme de la prise d'oméga-3 soit connu Taghizadeh et al, 2017 [32], l'association entre oméga-3 et *clostridium XVIII* n'a jamais été démontrée jusqu'à aujourd'hui (cf. exemple 2). Elle est même surprenante par rapport aux résultats publiés sur l'effet des omega-3 sur le microbiote intestinal (Fu et al. 2021).

Les présents inventeurs proposent donc d'administrer, par voie orale, une quantité efficace d'au moins un, de préférence deux, de préférence trois, voire quatre de ces nutriments (choisis parmi le bêta-carotène, les oméga-3, le galactomannane et le glucomannane), chez les patients ayant une perte bactérienne au niveau de ces genres bactériens d'intérêt (*Roseburia, Clostridium XVIII, Blautia, et*/*ou Faecalibacterium*), notamment chez les patients atteints de la maladie de Parkinson.

Idéalement, ces nutriments pourront être administrés sous forme de complément alimentaire ou de prébiotiques, comme décrit ci-dessous.

En outre, la prise de butyrate de sodium semble bénéfique chez les personnes atteintes de la maladie de Parkinson. Il est également avantageux de moduler la quantité de bactéries productrices de butyrate en modifiant leur alimentation, par exemple en leur administrant des aliments / nutriments tels que notamment des complexes Chitin-glucan, des glucanes b-1,3/1,6-D, des grains d'orge , ou de blé (Cantu-Jungles TM et al, 2019 [33]). Ces aliments / nutriments ne font pas partie de cette invention.

Néanmoins, les présents inventeurs proposent d'ajouter, dans le complément alimentaire de l'invention, en association avec les nutriments décrits ci-dessus, une quantité efficace de butyrate de sodium.

En résumé, les nutriments suivants peuvent être avantageusement utilisés pour augmenter l'abondance relative des bactéries présentant une faible abondance chez les patients souffrant de la maladie de Parkinson :

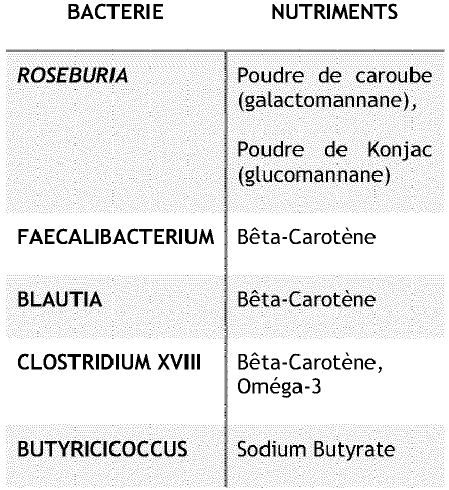

Dans un premier aspect, la présente invention concerne donc une formulation orale, par exemple un complément alimentaire ou une composition prébiotique, comprenant une quantité efficace d'au moins un nutriment choisi parmi : le bêta-carotène, le glucomannane, le galactomannane, les oméga-3 et le butyrate.

De préférence, cette formulation contient au moins deux nutriments de cette liste, par exemple du bêta-carotène et du glucomannane, du bêta-carotène et du galactomannane; du bêta-carotène et du butyrate; du bêta-carotène et des oméga-3; du glucomannane et du galactomannane; du glucomannane et du butyrate; du glucomannane et des oméga-3; du galactomannane et du butyrate; du galactomannane et des oméga-3; ou encore du butyrate et des oméga-3.

De préférence, cette formulation contient au moins trois nutriments de cette liste, par exemple du bêta-carotène, du glucomannane, et du galactomannane; du bêta-carotène, du glucomannane et du butyrate; du bêta-carotène, du glucomannane et des oméga-3; du glucomannane, du galactomannane et du butyrate; du glucomannane, du galactomannane et des oméga-3; du galactomannane, du butyrate et des oméga-3.

De préférence, cette formulation contient au moins quatre nutriments de cette liste, par exemple: du bêta-carotène, du glucomannane, du galactomannane, et du butyrate; du bêta-carotène, du glucomannane, du galactomannane, et des oméga-3; du glucomannane, du galactomannane, du butyrate, et des oméga-3; du bêta-carotène, du galactomannane, du butyrate, et des oméga-3.

De manière encore plus préférée, la formulation de l'invention contient les 5 nutriments de cette liste, à savoir du bêta-carotène, du glucomannane, du galactomannane, du butyrate, et des oméga-3.

Il est en outre possible d'incorporer des vitamines dans cette formulation.

Cette formulation peut être appelée « complément alimentaire » ou « formulation nutraceutique » ou « composition prébiotique » ou « composition nutraceutique » selon l'invention. Elle est destinée à améliorer la qualité de vie de sujets présentant une faible abondance relative des genres bactériens *Roseburia, Blautia, Faecalibacterium, Butyricicoccus* et/ou *Clostridium_XVIII,* plus précisément une moindre abondance des genres *Blautia, Faecalibacterium,* ou *Roseburia,* notamment chez des patients souffrant de la maladie de Parkinson.

La présente invention vise donc un procédé permettant d'améliorer la qualité de vie des patients atteints de la maladie de Parkinson, ledit procédé comprenant l'administration à ces patients de la formulation de l'invention. Cette formulation est destinée à favoriser le développement des bactéries des genres *Roseburia, Blautia, Faecalibacterium, Butyricicoccus* et/ou *Clostridium_XVIII* dans le microbiote desdits patients. En tant qu'agent prébiotique, elle n'a pas nécessairement de vocation médicale.

Une telle formulation orale pourra, lorsqu'elle est administrée régulièrement, permettre de réduire les troubles liés au déséquilibre de leur flore intestinale, permettant ainsi d'améliorer leur qualité de vie chez ces sujets.

La présente invention vise également un procédé pour augmenter l'abondance relative des genres bactériens *Roseburia, Blautia, Faecalibacterium, Butyricicoccus* et/ou *Clostridium_XVIII* au sein du microbiote intestinal d'un individu, caractérisé en ce qu'il comprend l'administration d'une quantité efficace de l'un des cinq nutriments identifiés par les inventeurs, par voie orale, à cet individu.

Plus précisément, ledit procédé permet d'augmenter l'abondance relative des genres bactériens *Blautia* ou *Faecalibacterium* ou *Clostridium XVIII* chez les patients présentant une abondance relative de l'un de ces genres bactériens diminuée. En particulier, ledit procédé permet d'augmenter l'abondance précisément de ces bactéries chez les patients souffrant de la maladie de Parkinson. Comme démontré dans les exemples ci-dessous, il suffit dans ce cas d'administrer une formulation contenant uniquement du bêta-carotène.

Ainsi, la présente demande vise l'utilisation de bêta-carotène pour augmenter l'abondance relative des genres bactériens *Blautia* ou *Faecalibacterium* ou *Clostridium XVIII* chez les patients présentant une abondance relative de l'un de ces genres bactériens diminuée, notamment pour améliorer le bien-être des patients souffrant de la maladie de Parkinson.

Plus précisément, ledit procédé permet d'augmenter l'abondance relative des bactéries du genre *Roseburia* chez les patients présentant une abondance relative de ces bactéries diminuée. En particulier, ledit procédé permet d'augmenter l'abondance précisément de ce type de bactéries chez les patients souffrant de la maladie de Parkinson. Comme démontré dans les exemples ci-dessous, il suffit dans ce cas d'administrer une formulation contenant uniquement du glucomannane ou du galactomannane.

Ainsi, la présente demande vise l'utilisation de glucomannane et/ou du galactomannane pour augmenter l'abondance relative de bactéries du genre *Roseburia* chez les patients présentant une faible abondance relative de ces bactéries, notamment pour améliorer le bien-être des patients souffrant de la maladie de Parkinson.

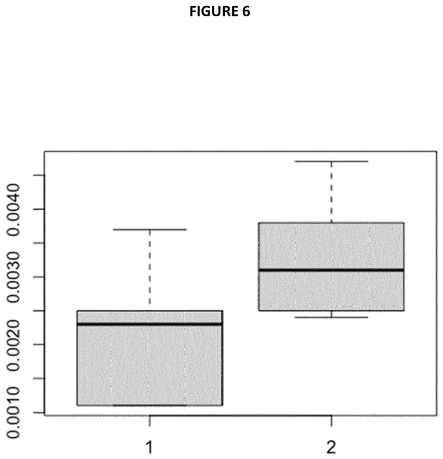

Le galactomannane présentant également une activation de la production de dopamine (cf. exemple 3 et figure 6), la présente demande vise l'utilisation de ce nutriment particulier pour améliorer par ce biais le bien-être des patients souffrant de la maladie de Parkinson.

Le terme « abondance d'un genre bactérien » désigne ici la quantité des bactéries dudit genre bactérien, identifié grâce à des marqueurs géniques spécifiques des bactéries dudit genre bactérien. Cette quantité peut être mesurée par tout moyen connu dans l'art, notamment en analysant l'ADN présent dans un échantillon de selles prélevé chez un sujet, par exemple par PCR quantitative, par séquençage, en utilisant des puces à ARN/ADN, ou encore en utilisant des anticorps capables de discriminer différents genres bactériens. Il est préférable de mesurer la quantité des gènes codant pour l'ARN ribosomique 16S (ARNr 16S) qui est très spécifique de chaque genre bactérien et est couramment utilisé à cet effet [22].

L'«abondance relative» d'un genre bactérien désigne la quantité des bactéries dudit genre par rapport à la quantité totale de toutes les bactéries présentes dans l'intestin de l'individu ou dans un échantillon de selles de cet individu. Il correspond au rapport entre la quantité de bactéries d'un genre bactérien donné dans un échantillon (de préférence un échantillon de selle), divisé par la quantité totale de bactéries présentes dans ledit échantillon.

Le procédé de l'invention permet d'augmenter ladite abondance relative d'au moins 50%, de préférence d'au moins 100%, de manière encore plus préférée de 300%, par rapport à l'abondance relative initiale chez le sujet étudié, avant administration de la composition de l'invention. Cette augmentation peut par exemple se détecter en mesurant la proportion de bactéries *Roseburia, Blautia, Faecalibacterium, Butyricicoccus* et/ou *Clostridium_XVIII* dans les selles des sujets, avant administration de la composition, et après administration de la composition pendant au moins quatre semaines.

Bien que n'importe quel moyen puisse être utilisé pour mesurer cette augmentation, la technologie utilisant la quantification des gènes codant pour l'ARNr 16S est un moyen privilégié.

Le bêta-carotène (ou provitamine A) est un antioxydant de la famille des caroténoïdes, principalement retrouvé dans les produits d'origine végétale tels que la patate douce (cuite), la carotte (crue), la tomate, les épinards (cuits), le potiron, la framboise, la pastèque, l'abricot, la mangue ou encore le pruneau.

Il est possible de produire du bêta-carotène par différentes méthodes bien connues de l'homme du métier (Bogacz-Radomska et al, 2018 [23]). Ces méthodes sont par exemple physico-chimiques (par extraction à partir d'une matière végétale riche en caroténoïdes), chimique (obtention de bêta-carotène synthétique par exemple par réaction de Wittig), ou par biosynthèse microbiologique (notamment à partir d'algues ou de levures synthétisant du bêta-carotène).

Une « quantité efficace de bêta-carotène » est, dans le cadre de l'invention, définie comme toute quantité de bêta-carotène permettant d'augmenter l'abondance relative des bactéries de genre *Roseburia, Blautia, Faecalibacterium, Butyricicoccus* et/ou *Clostridium_XVIII* et en particulier des genres *Blautia* et/ou *Faecalibacterium* d'un facteur 0.5, 1 ou 3, chez des patients présentant une faible abondance bactérienne, notamment chez des patients souffrant de la maladie de Parkinson.

Lorsque le bêta-carotène est administré à l'individu sous forme d'un complément alimentaire, en poudre ou en pilule, une telle quantité efficace est par exemple comprise entre 600µg et 1500µg, de préférence entre 1000µg et 1500µg, encore plus préférentiellement entre 1000 et 1300 µg de bêta-carotène par jour. Cette quantité sera additionnée à la quantité ingurgitée par ailleurs par l'individu au cours de ses prises alimentaires.

Ledit bêta-carotène peut également être administré à l'individu par le biais d'un apport alimentaire spécifiquement conçu pour être enrichi en bêta-carotène. Dans ce cas, les repas dudit individu seront enrichis en aliments riches en bêta-carotène, par exemple choisis parmi : la patate douce (cuite), la carotte, la tomate, les épinards, le potiron, la framboise, la pastèque, l'abricot, la mangue ou encore le pruneau.

Lorsque le bêta-carotène est administré à l'individu par le biais d'un apport alimentaire spécifiquement conçu pour être enrichi en bêta-carotène, ladite quantité efficace en bêta-carotène est comprise entre 6 et 15 mg par jour, de préférence entre 10 et 15 mg par jour, de manière encore plus préférée entre 12 et 15mg par jour.

Dans un mode de réalisation préféré, le bêta-carotène est administré sous forme de compléments alimentaires, par exemple en poudre ou en gélule. Il est possible par exemple d'utiliser de la poudre de carotte, qui est naturellement riche en bêta-carotène. Dans ce cas, il est préférable d'administrer entre 6 et 15 mg de poudre de carotte par jour.

Par « améliorer la qualité de vie », on entend ici réduire des symptômes gastro-intestinaux tels que la constipation, ou encore améliorer le confort digestif de ces patients.

Dans un autre aspect, la présente invention vise l'utilisation du bêta-carotène, ou l'utilisation d'une formulation orale (composition nutraceutique ou complément alimentaire) contenant une quantité efficace de bêta-carotène, pour améliorer la qualité de vie de patients atteints de la maladie de Parkinson. De préférence, ladite formulation orale est telle que définie ci-dessus. En particulier, elle contient soit du bêta-carotène seul, soit du bêta-carotène associé avec une quantité efficace d'oméga-3, de galactomannane, du glucomannane et/ou du butyrate.

Le galactomannane est un polymère linéaire composé d'une chaîne de monomères de mannose ((1,4)-beta-D-mannopyranose) auxquelles sont ramifiés par un pont 1-6 une unité de galactose. On le trouve dans les graines des végétaux, où il sert de réserve de sucre lors de la germination. Il est abondant dans l'albumen de graines de légumineuses, telle que *Cyamopsis tetragonoloba, Caesalpinia spinosa* et *Ceratonia siliqua.* Les galactomannanes utilisables dans le cadre de l'invention sont ceux approuvés et déjà utilisés dans l'agro-alimentaire. On peut citer par exemple les gommes naturelles : gomme de guar (E412), gomme tara (E417) et gomme de caroube (E410).

Les résultats obtenus dans l'exemple 3 démontrent pour la première fois que le galactomannane, par l'ajout de poudre de caroube, agit positivement sur la sécrétion de dopamine et sur l'abondance relative de bactéries *Roseburia.* Dans un autre aspect, la présente invention vise donc l'utilisation du galactomannane, ou l'utilisation d'une formulation orale (composition prébiotique, nutraceutique ou complément alimentaire) contenant une quantité efficace de galactomannane, pour améliorer la qualité de vie de patients atteints de la maladie de Parkinson. De préférence, ladite formulation orale est telle que définie ci-dessus. En particulier, elle contient soit du galactomannane seul, soit du galactomannane associé avec une quantité efficace d'oméga-3, de bêta-carotène, de glucomannane et/ou de butyrate.

La poudre de caroube est issue du caroubier, un arbre méditerranéen. La poudre est obtenue par broyage de l'écorce du fruit uniquement. Elle est riche en galactomannane. De manière avantageuse, la formulation de l'invention comprend de la poudre de caroube qui contient une quantité efficace de galactomannane. Lorsque la poudre de caroube est utilisée dans la formulation de l'invention, une telle quantité efficace de galactomannane est atteinte en administrant par exemple entre 5 à 30g par jour de poudre de caroube.

Le konjac, ou *amorphophalus konjac,* est un légume provenant d'Asie du Sud-Est. Il pousse en forêt au Vietnam, en Chine, en Indonésie, Corée du Sud et au Japon. Ses racines contiennent du glucomannane, un gélifiant très efficace qui « tapisse » l'estomac lorsqu'il est ingéré. Il existe des compléments alimentaires contenant du konjac, utilisé comme « coupe-faim ». Il a récemment été démontré que cet ingrédient augmente la quantité de bactéries de genre *Roseburia* (La Rosa et al, 2019 [30]).

Dans un autre aspect, la présente invention vise l'utilisation du glucomannane, notamment du glucomannane de konjac, ou l'utilisation d'une formulation orale (composition nutraceutique ou complément alimentaire) contenant une quantité efficace de glucomannane, pour améliorer la qualité de vie de patients atteints de la maladie de Parkinson. De préférence, ladite formulation orale est telle que définie ci-dessus. En particulier, elle contient soit du glucomannane seul, soit du glucomannane associé avec une quantité efficace d'oméga-3, de galactomannane, du bêta-carotène et/ou du butyrate.

Lorsque le glucomannane de konjac est administré à l'individu sous forme d'un complément alimentaire, en poudre ou en pilule, une telle quantité efficace est par exemple comprise 1 et 5g par jour.

Si la formulation de l'invention contient des oméga-3, ces oméga-3 sont de préférence l'acide docohexaènoïque (DHA) ou l'acide eïcosapentaènoïque (EPA). Dans ce cas, il est recommandé d'administrer aux patients environ 250mg/jour de DHA et/ou d'EPA, sans dépasser 2g par jour.

Dans la formulation de l'invention, les nutriments peuvent être ajoutés de manière isolée, ou ils peuvent être apportés par des aliments qui contiennent ces nutriments en grande quantité. Par exemple, comme mentionné ci-dessus, il est possible d'utiliser de la poudre de caroube qui est riche en galactomannane, ou de la poudre de konjac, qui est riche en glucomannane. Il est également possible d'utiliser de la poudre de carotte riche en caroténoïdes, notamment en bêta-carotène. Ces poudres sont disponibles dans le commerce, déjà proposées en tant que compléments alimentaires, principalement à des fins cosmétiques pour améliorer l'aspect de la peau.

Dans un mode de réalisation particulier, la formulation orale de l'invention contient une quantité efficace de poudre de carotte et/ou une quantité efficace de poudre de konjac et/ou une quantité efficace de poudre de caroube. Les quantités en question sont telles que proposées plus haut.

Dans un mode de réalisation très particulier, la formulation orale de l'invention comprend de la poudre de carotte, de la poudre de konjac, et de la poudre de caroube.

Dans un autre aspect, la présente invention concerne l'utilisation thérapeutique de la formulation de l'invention, afin de prévenir l'apparition de la maladie de Parkinson, diminuer son évolution, et réduire la posologie quotidienne des médicaments précités. Dans cet aspect, l'invention vise donc également la formulation de l'invention, pour son utilisation pour prévenir ou traiter les symptômes de la maladie de Parkinson, et/ou d'adapter les traitements administrés aux patients souffrant de la maladie de Parkinson.

De manière plus générale, il est possible d'utiliser la formulation de l'invention pour prévenir ou traiter ou adapter les traitements de toutes les maladies dues ou associées à une faible abondance des bactéries *Roseburia, Blautia, Faecalibacterium, Butyricicoccus* et/ou *Clostridium_XVIII* dans le microbiote intestinal. Ces maladies sont notamment des maladies neurodégénératives telles que la maladie d'Alzheimer, la maladie de Charcot, la sclérose latérale amyotrophique, la maladie de Huntington, ou encore la maladie à corps de Lewy, dont l'étiologie peut être liée à une faible abondance dans un des genres bactériens identifiés par les inventeurs pour la maladie de Parkinson.

En d'autres termes, il est possible d'utiliser du bêta-carotène, du glucomannane, et/ou du galactomannane, et éventuellement des oméga-3 et/ou du butyrate, pour préparer un médicament destiné à prévenir ou traiter les symptômes des maladies neurodégénératives chez des patients présentant une faible abondance des bactéries du genre *Roseburia, Blautia, Faecalibacterium, Butyricicoccus* et/ou *Clostridium_XVIII* dans leur microbiote intestinal.

Dans un autre aspect, la présente invention vise une méthode permettant d'établir un régime alimentaire adapté à des individus en fonction de leur niveau d'abondance des 5 genres bactériens identifiés ci-dessus. Cette méthode comprend les étapes suivantes :
a) déterminer le niveau d'abondance ou d'abondance relative des 5 genres bactériens,
b) en fonction du niveau, recommander un régime alimentaire enrichi en l'un des nutriments défini ci-dessus, pour les individus présentant un microbiote dans lequel les genres bactériens *Roseburia, Blautia, Faecalibacterium, Butyricicoccus* et/ou *Clostridium_XVIII* et plus particulièrement les genres bactériens *Blautia* et *Faecalibacterium* sont diminués.

En application des résultats de la présente demande, le régime alimentaire pourra être enrichi en :
- bêta-carotène lorsque l'individu présente une carence en bactéries du genre *Blautia* ou *Faecalibacterium* ou *Clostridium XVIII,*
- glucomannane, par exemple en poudre de konjac lorsque l'individu présente une carence en bactéries du genre *Roseburia,*
- galactomannane, par exemple en poudre de caroube, lorsque l'individu présente une carence en bactéries du genre *Roseburia,*
- omega-3, par exemple en DHA ou en EPA, lorsque l'individu présente une carence en bactéries du genre *Clostridium XVIII,*
- butyrate lorsque l'individu présente une carence en bactéries du genre *Butyricicoccus.*

L'homme du métier saura identifier les aliments à utiliser, ainsi que leurs quantités, en fonction des informations fournies dans la présente invention, et des tableaux présentant les apports nutritionnels par portion d'aliments, notamment le tableau de composition alimentaire française de l'ANSES.

### DESCRIPTION DES FIGURES

La figure 1 représente la distribution du genre bactérien *Butyricicoccus* chez les patients sains (HC) et souffrant de la maladie de Parkinson(PD), telle que détectée et quantifiée dans les cohortes décrites par Aho et al, Hill et al, Keshavarzian et al, Petrov et al et Pietrucci et al.
La figure 2 représente la distribution du genre bactérien *Roseburia* chez les patients sains (HC) et souffrant de la maladie de Parkinson (PD), telle que détectée et quantifiée dans les cohortes décrites par Aho et al, Hill et al, Keshavarzian et al, Petrov et al et Pietrucci et al.
La figure 3 représente la distribution du genre bactérien *Blautia* chez les patients sains (HC) et souffrant de la maladie de Parkinson (PD) , telle que détectée et quantifiée dans les cohortes décrites par Aho et al, Hill et al, Keshavarzian et al, Petrov et al et Pietrucci et al.
La figure 4 représente la distribution du genre bactérien *Clostridium XVIII* chez les patients sains (HC) et souffrant de la maladie de Parkinson (PD), telle que détectée et quantifiée dans les cohortes décrites par Aho et al, Hill et al, Keshavarzian et al, Petrov et al et Pietrucci et al.
La figure 5 représente la distribution du genre bactérien *Faecalibacterium* chez les patients sains (HC) et souffrant de la maladie de Parkinson (PD) , telle que détectée et quantifiée dans les cohortes décrites par Aho et al, Hill et al, Keshavarzian et al, Petrov et al et Pietrucci et al.
La figure 6 représente les niveaux de dopamine détectés dans les échantillons du groupe sans supplémentation (1, à gauche) et avec administration de galactomannane (2, colonne droite). Une nette augmentation en dopamine est visible lorsque le galactomannane est ajouté.

### EXEMPLES

### Exemple 1

Une méta-analyse a été réalisée sur l'abondance relative des genres bactériens afin d'identifier les marqueurs associés à la maladie de Parkinson au sein d'une population âgée, nord-américaine et européenne. Cette méta-analyse a mis en évidence une signature de 6 genres bactériens, à savoir *Akkermansia,* le seul genre significativement augmenté chez les Parkinsons en comparaison avec les contrôles, ainsi que *Blautia, Butyricicoccus, Clostridium XVIII, Faecalibacterium* et *Roseburia,* tous significativement diminués chez les Parkinsons.

### 1/ Matériel et méthodes

Après une recherche exhaustive de la littérature pour identifier des articles reportant des résultats originaux comparant le microbiote de patients Parkinsoniens au microbiote intestinal de contrôles, 7 études ont été identifiées, pour lesquelles les données brutes de séquençage étaient accessibles.

Les fichiers Fastq de chaque étude ont été téléchargés depuis les bases de données, notamment la base de données SRA (Sequencing Read Archive). Certaines données n'étant pas disponibles, nous avons adressé une demande aux auteurs pour récupérer les données de séquençage et/ou les données cliniques associées. Les échantillons "non-fécaux" (par exemple les "mock samples") ont été supprimés de l'analyse bioinformatique. Pour chaque étude, les fichiers fastq ont été utilisés grâce au pipeline propriétaire de Luxia Scientific, adaptée de QIIME2 pipeline (version 2019.10) (Bolyen et al., 2019 [24]). La qualité des séquences a été analysée avec l'outil FastqC. Les séquences de mauvaise qualité ont été supprimées. La taille des séquences variait en fonction des études, mais également au sein de chaque étude.

L'annotation taxonomique des genres bactériens à partir des séquences représentatives a été réalisée à l'aide de la base de données RDP (version 11) (Cole et al., 2014 [25]). Une matrice contenant les comptes bruts par échantillon et genre bactérien a été obtenue. De cette matrice creuse, les valeurs à moins de 5 comptes (<5) ont été imputées à 0 pour réduire le bruit de fond. Puis l'abondance relative des genres bactériens a été estimée en divisant les comptes de chaque genre par la profondeur de séquençage initiale. Pour chaque étude, les genres bactériens ont été filtrés afin de concentrer l'analyse statistique uniquement sur les genres prévalents. Ainsi, un genre bactérien a été conservé s'il possède une prévalence d'au moins 35% dans l'un des deux groupes (PD, HC), c'est-à-dire qu'il doit être quantifié dans aux moins 35% des échantillons d'un groupe d'analyse. Les genres bactériens sans annotation précise ont été exclus car le rapprochement entre les études a été réalisé sur la base de l'annotation taxonomique. Cette étape de filtre (imputation, prévalence, annotation) des genres bactériens a été réalisée pour chaque étude indépendamment.

Après le traitement bioinformatique des données, et un contrôle qualité des données traitées, deux études ont été exclues de la présente analyse et n'ont été conservés que les échantillons de bonne qualité. Ces études caractérisent le microbiote intestinal par métagénomique 16S de populations nord-américaines et européennes.

Pour réaliser la méta-analyse, nous avons utilisé la fonction metagen du package « R meta », qui standardise les estimations pour ensuite réaliser une méta-analyse par inverse de la variance. Un modèle « random » a été retenu, pour prendre en compte la variance de chaque étude. La fonction metagen revoie les estimateurs meta-analysés, avec les intervalles de confiance et une p-valeur pour chaque genre bactérien. Pour être méta-analysé, un genre bactérien doit être quantifié dans au moins trois études. Les p-valeurs des estimateurs méta-analysés ont ensuite été corrigées par Benjamini-Hochberg [26].

Une évaluation de la signature a été réalisée. Pour ce faire, pour chaque étude, un modèle de régression logistique a été ajusté avec les genres bactériens sélectionnées en variables explicatives et la classification des patients (Parkinson vs. Contrôle) en variable de réponse. L'évaluation a été faite en LOOCV (leave-one-out cross-validation), une validation croisée itérative qui consiste à mettre un échantillon de côté, apprendre les paramètres de l'algorithme sur les autres échantillons afin de prédire le statut de l'échantillon isolé, et ce itérativement pour l'ensemble des échantillons. Basé sur les prédictions, pour chaque étude, une courbe ROC a été produire et l'AUC (aire sous la courbe) a été calculé avec un intervalle de confiance à 95%. Il est important de noter que les abondances relatives ne sont pas directement comparables d'une étude à l'autre, ainsi que les modèles prédictifs ont été construits indépendamment pour chaque étude.

Les analyses statistiques ont été réalisées avec le logiciel R (https://www.r-project.org/) (version 3.6.3). Les scripts R ont été compilés avec le package knitr et sauvegardés sous format HTML.

### 2/ Résultats

**Le TABLEAU 1 ci-dessous donne les détails concernant les 7 études qui ont été sélectionnées et la longueur des séquences pour chaque étude.**

| **Etude** | **Longueur utilisée pour la quantification des espèces (nts)** | **Médiane des longueurs de séquences sur 10 000 tirages (nts)** | **[2ème - 98ème] percentiles des longueurs de sequences sur 10 000 tirages (nts)** | **Variabilité dans la longueur des séquences** |
|---|---|---|---|---|
| Heintz- Buschart [27] | 150 | 573 | [233 - 584] | +++ |
| Hill-Burns [11] | 148 | 150 | [150 - 150] | - |
| Hopfner [28] | 300 | 315 | [304 - 343] | + |
| Keshavarzian [13] | 250 | 253 | [207 - 253] | + |
| Petrov [16] | 230 | 134 | [25 - 234] | +++ |
| Aho [1] | 400 | 432 | [402 - 473] | + |
| Pietrucci [15] | 400 | 408 | [402 - 427] | + |

Pour certaines études comme Heintz-Buschart ou encore Petrov, la longueur des séquences obtenues dans les fichiers de séquençage varie beaucoup. Par exemple, pour Petrov, la médiane des longueurs de séquence est de 134 nucléotides (nts) mais varie entre 25 (2ème percentile) et 234 (98ème percentile). La distribution des longueurs de séquences ne suit pas une loi prédéterminée. Logiquement, avec un pipeline standardisé, la longueur devrait être constante (ou très peu varier) pour chaque échantillon, comme pour Hill-Burns par exemple. Au final, pour chaque étude, nous avons utilisé une longueur prédéfinie, en trouvant un compromis pour ne pas perdre trop d'échantillons, tout en gardant une longueur aussi grande que possible.

Comme indiqué dans la partie méthode, avant de calculer la proportion de chaque genre par échantillon, à partir de la table de comptage au niveau des genres bactériens, nous avons imputé à 0 les genres avec moins de 5 comptages par échantillon (<5). Les genres trop faiblement détectés peuvent être non-spécifiques, et ajouter de la variabilité dans l'analyse. En proportion, sur l'ensemble des comptages, cela correspond à environ 2% à 6% de l'ensemble des données qui sont imputées à 0. Les matrices de comptage ont la particularité d'être creuses. Après imputation des données, la proportion de valeur à 0 est assez comparable d'une étude à l'autre, allant de 70% à 85% environ.

Afin de réaliser la méta-analyse, les genres bactériens sont filtrés pour deux raisons principales :
- Certains genres sont très peu prévalents - or seules les espèces assez prévalentes et surtout quantifiables nous intéressent pour la méta-analyse afin d'avoir la signature la plus universelle possible,
- Certains genres peuvent avoir une annotation taxonomique insuffisante ce qui ne permettra pas de faire une comparaison entre étude, qui est basée sur l'annotation taxonomique.

Dans chaque étude, un genre bactérien est conservé s'il a une prévalence d'au moins 35% dans l'un des deux groupes (PD = patients souffrant de la maladie de Parkinson, HC = sujets sains), c'est-à-dire qu'il doit être quantifié dans aux moins 35% des échantillons d'un groupe d'analyse. Entre 63% et 82% des genres sont ainsi éliminés de l'analyse.

Enfin, les genres qui sont sans annotation précise sont également supprimés de l'analyse. Cela correspond approximativement à 16% - 25% des genres restants après le filtre sur la prévalence. Au final, par étude, une soixante de genres a été étudiée dans la méta-analyse.

Le résultat de la méta-analyse pour les six genres significatifs est reporté dans le tableau 2 ci-dessous. Les estimations méta-analysées peuvent se traduire en odds-ratios. Cet odd-ratio peut se comprendre, par exemple pour *Akkermansia,* comme suit : les patients Parkinson ont, à travers les différentes études, 1.34 fois plus de risque d'avoir *Akkermansia* augmentée que les contrôles. Pour les genres diminués chez les patients Parkinson, comme par exemple *Roseburia,* les patients Parkinson ont 1.64 (1/0.61) fois plus de risque d'avoir *Roseburia* diminuée que les contrôles.

**Tableau 2 : résultat de la méta-analyse**

| **Genres** | **Estimations meta-analysés** | **Odd-ratio** | **p-valeur ajustée** | **Quantité chez patients PD** |
|---|---|---|---|---|
| Roseburia | -0.488 | 0.61 | 0 | Diminuée |
| Blautia | -0.339 | 0.71 | 0 | Diminuée |
| Faecalibacterium | -0.372 | 0.63 | 0.008 | Diminuée |
| Akkermansia | 0.294 | 1.34 | 0.008 | Augmentée |
| Butyricicoccus | -0.204 | 0.81 | 0.009 | Diminuée |
| Clostridium_XVIII | -0.2 | 0.81 | 0.039 | Diminuée |

Cette méta-analyse a mis en évidence une signature de 6 genres bactériens, à savoir *Akkermansia,* le seul genre significativement augmenté chez les patients Parkinson en comparaison avec les contrôles, ainsi que *Blautia, Butyricicoccus, Clostridium XVIII, Faecalibacterium* et *Roseburia,* tous significativement diminués chez les patients Parkinson. Le genre *Akkermansia* possède à ce jour une seule espèce caractérisée et connue dans le microbiote intestinal humain, *Akkermansia muciniphila.* Cette espèce est généralement associée à un bon phénotype clinique car elle se nourrit principalement de mucine, signe d'un intestin sain, son augmentation chez les Patients Parkinson n'est à ce jour pas expliquée. Les genres bactériens diminués sont tous des *Clostridiales* (Firmicutes) et sont principalement associés à des fonctions anti-inflammatoires via la production de butyrate. La technologie employée ne nous permet pas d'avoir une caractérisation précise au niveau de l'espèce, cependant à ce jour, le genre *Faecalibacterium* est caractérisé par une seule espèce, *Faecalibacterium praunsitzii,* et il en est de même pour *Butyricicoccus* avec l'espèce *Butyricicoccus pullicaecorum.* Concernant les autres genres augmentés chez les patients Parkinson et mis en avant dans cette méta-analyse, notons que *Roseburia* et *Blautia* ont principalement deux espèces caractérisées dans l'intestin, respectivement *Roseburia intestinalis, Roseburia faecis, Blautia luti et Blautia wexlerae,* quand *Clostridium XVIII* n'a pas d'espèces très bien caractérisées pour le moment. *Roseburia* est également associée à des fonctions de production de butyrate.

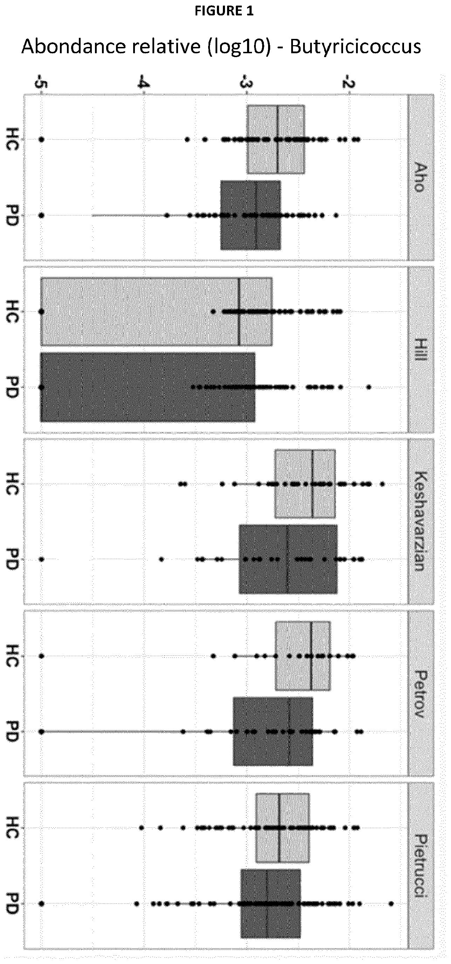

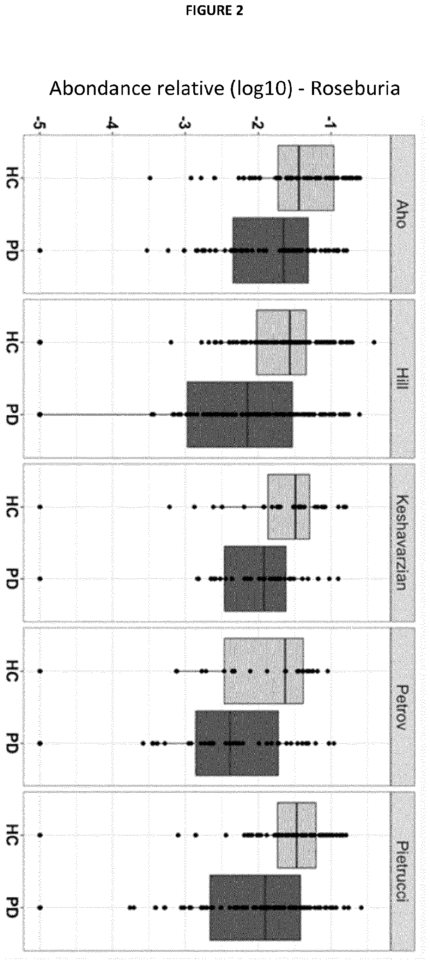

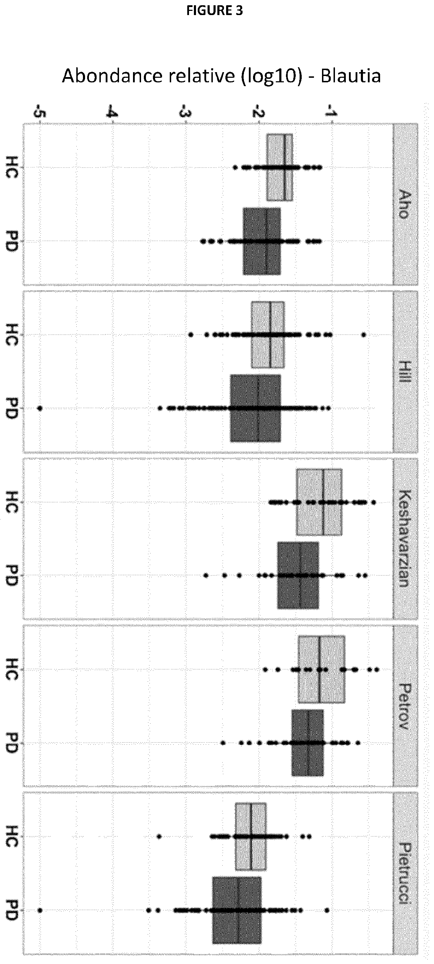

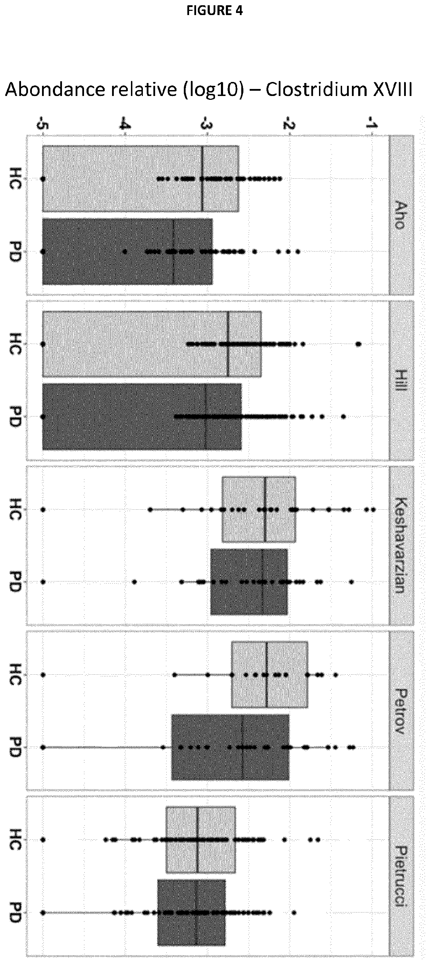

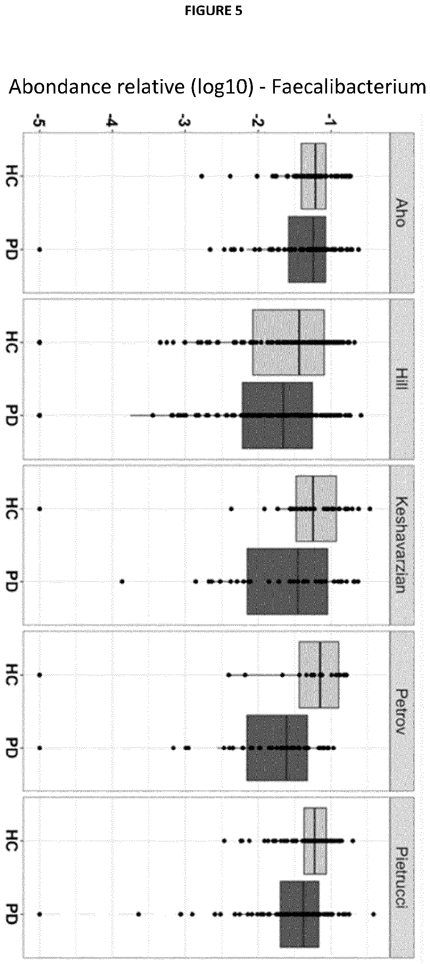

Les figures 1 à 5 montrent les distributions des genres bactériens associés avec la maladie de Parkinson pour l'ensemble des cohortes dans lesquelles ils sont détectés et quantifiés. L'abondance relative des genres bactériens est représentée en base log10.

Cette méta-analyse démontre que malgré l'hétérogénéité des données (différentes plateformes, différentes populations), le microbiote des patients Parkinson est altéré sur une même base de marqueurs bactériens dont les modulations sont cohérentes entre les différentes cohortes.

Récemment, une méta-analyse réalisée par une équipe japonaise (Nishiwaki et al. 2020 [15]) a montré que les genres *Akkermansia, Catabacter, Hungatella* et *Coprobacillus* sont augmentés chez patients Parkinson en comparaison avec une population du même âge, alors que les genres *Roseburia, Faecalibacterium, Lachnospiraceae ND3007* et *Lachnospiraceae UCG-004* sont diminués chez ces mêmes patients. En reproduisant la même méta-analyse, mais avec des critères qualités plus strictes et une base de données taxonomiques différentes, nous avons montré que *Akkermansia, Faecalibacterium* et *Roseburia* sont associés à la maladie, mais également les genres *Butyricicoccus, Clostridium_XVIII* ainsi que *Blautia.* En revanche, notre base de données n'a pas permis d'identifier les autres espèces caractérisées par cette équipe japonaise.

### Exemple 2

Une étude exploratoire a été réalisée pour mettre en évidence les éventuelles associations entre les apports en aliments/nutriments des individus et les cinq bactéries d'intérêt identifiées dans l'exemple 1 *(Faecalibacterium, Blautia, Roseburia, Butyricicoccus, Clostridium-XVIII).*

L'objectif principal était d'identifier la meilleure composition possible d'un complément alimentaire à développer afin d'améliorer le bien-être d'individus ayant une diminution au niveau de ces cinq bactéries d'intérêt, et notamment, pour les patients atteints de la maladie de Parkinson.

Les tests statistiques suivants ont été réalisés :
- Corrélations non paramétriques de Spearman entre les apports en nutriments calculés et chacune des cinq bactéries d'intérêt.
- Corrélations non paramétriques de Spearman entre les fréquences de consommation des aliments du questionnaire alimentaire et chacune des cinq bactéries d'intérêt.
- Régressions ordinales entre les fréquences de consommation des aliments du questionnaire alimentaire et chacune des cinq bactéries d'intérêt.

### 1 / Matériel et méthode

### 1.1. Principaux logiciels utilisés :

| **Nom** | **Version** | **Objet** |
|---|---|---|
| R | 3.6.2 | Logiciel permettant d'effectuer les calculs statistiques, les tables et les figures de ce rapport. |
| R Studio | 1.2.5033 | Interface d'utilisation de R et de Rmarkdown (qui permet la rédaction de rapports reproductibles et dynamiques sous R). |

### 1.2. Recueil des réponses au questionnaire alimentaire

Après avoir créé leur compte personnel sur le site internet de la société de Luxia Scientific, les clients peuvent remplir de manière volontaire un questionnaire alimentaire. Ce questionnaire possède neuf sous-groupes de questions, chacun constituant une catégorie d'aliments. À l'intérieur de ces sous-groupes, une liste complète d'aliments est fournie et donne aux personnes la possibilité de choisir la fréquence à laquelle elles ont consommé chaque aliment possible au cours du dernier mois dans une portion spécifiée.

L'export des questionnaires alimentaires complétés à 100% en date du 1er février 2021 a permis de recueillir 288 questionnaires.

Une étape supplémentaire de filtration des données est ensuite réalisée : à partir des questionnaires complètements remplis, on ne conserve que les réponses des questions interprétables en valeurs nutritionnelles. C'est-à-dire que l'on retire les questions sur les habitudes alimentaires des consommateurs (lieu d'achat des aliments, habitudes de cuisson/consommation, etc...).

Ainsi, on obtient le fichier des réponses au questionnaire pour 145 aliments pour 288 questionnaires complètement remplis.

### 1.3. Recueil des données nutritionnelles de la Table Ciqual

Un tableau des apports nutritionnels par portion d'aliments présents dans le questionnaire a été ensuite créé. Pour créer ce tableau, la Table de composition alimentaire française Ciqual de l'ANSES du 07/07/2020 (https://ciqual.anses.fr/) a été utilisée.

Ensuite, si plusieurs aliments de la Table Ciqual peuvent correspondre à un seul aliment du questionnaire, la priorité a été donnée, dès que possible, à l'aliment étiqueté "(aliment moyen)" comme l'apport en nutriments correspondant. Si plusieurs aliments du tableau Ciqual sont étiquetés "(aliment moyen)" et peuvent tous correspondre à un seul aliment du questionnaire, alors nous prenons tous ces aliments étiquetés "(aliment moyen)" pour calculer les moyennes de leurs apports en tant qu'apports en nutriments correspondants. À l'inverse, si aucun aliment étiqueté "(aliment moyen)" ne peut être trouvé dans la table Ciqual pour correspondre à un aliment du questionnaire, ont été pris dans la table Ciqual les apports de chaque variante de cet aliment spécifique qui est couramment consommée en France. Ensuite, la moyenne des apports nutritionnels de toutes ces variantes a été calculée, elle constitue l'apport correspondant pour cet aliment.

L'apport en omégas-3 a été calculé en additionnant les valeurs d'apports en acide alpha-linolénique (18:3 ; ALA), en acide eïcosapentaénoïque (20:5 ; EPA) et en acide docosahexaénoïque (22:6 ; DHA). L'apport en omégas-6 a été calculé en additionnant les valeurs d'apports en acide linoléique (18:2 ; ALA) et en acide arachidonique (20:4 ; ARA) qui sont les deux présents dans la Table Ciqual.

### 1.4. Recueil de données sur les polyphénols, l'amidon résistant, les FOS et les GOS

Comme la Table Ciqual ne fournit pas d'informations sur les apports en polyphénols des aliments, la base de données de Phenol-Explorer (version 3.6) a été utilisée pour récupérer uniquement les teneurs totales en polyphénols des aliments. Similairement aux aliments de la table Ciqual, la priorité a été donnée, dans la mesure du possible, à l'aliment qui correspond le mieux dans la base de données de Phenol-Explorer comme étant l'apport en polyphénols correspondant de l'aliment du questionnaire. Si plusieurs aliments de cette base de données peuvent être assimilés à un seul aliment du questionnaire, les apports en polyphénols de tous ces aliments correspondants dans la base de données ont été pris pour calculer la moyenne comme étant l'apport en polyphénols correspondant pour cet aliment dans le questionnaire. Si un aliment dans le questionnaire n'a pas son équivalent dans la base de données Phenol-Explorer, alors, lorsque cela est possible, l'apport en polyphénols de la variation la plus proche de cet aliment a été pris comme l'apport correspondant.

Pour la teneur en amidon résistant des aliments, la base de données nutritionnelle de la FSANZ (release 1) a été utilisée pour récupérer les teneurs totales en amidon résistant ('resistant starch'). Similairement aux aliments de la table Ciqual, la priorité a été donnée, dans la mesure du possible, à l'aliment qui correspond le mieux dans la base de données de la FSANZ comme étant l'apport en amidon résistant correspondant de l'aliment du questionnaire. Si plusieurs aliments de cette base de données peuvent être assimilés à un seul aliment du questionnaire, les apports en amidon résistant de tous ces aliments correspondants dans la base de données ont été pris pour calculer la moyenne comme étant l'apport en amidon résistant correspondant pour cet aliment dans le questionnaire. Si un aliment du questionnaire n'a pas son équivalent dans la base de données de la FSANZ, alors, lorsque cela est possible, l'apport en amidon résistant de la variation la plus proche de cet aliment a été pris comme l'apport correspondant.

La Table Ciqual ne fournit pas non plus toutes les teneurs en FOS et en GOS des aliments. Pour les FOS, nous avons donc récupéré les teneurs (g/100g fresh weight) présentes dans les deux tables de la publication Muir et al. [29] Pour les GOS, nous avons récupéré les teneurs présentes dans les trois tables de la publication Muir et al. [30] et additionné les teneurs des GOS détectés de chaque aliment que l'on retrouve dans notre questionnaire. De la même manière que pour les polyphénols, si plusieurs aliments des tables peuvent être assimilés à un seul aliment du questionnaire, les apports en FOS (respectivement GOS) de tous ces aliments correspondants ont été pris pour calculer la moyenne comme étant l'apport en FOS (respectivement GOS) correspondant pour cet aliment dans le questionnaire. Si un aliment du questionnaire n'a pas son équivalent dans les tables, alors, lorsque cela est possible, l'apport en FOS (respectivement GOS) de la variation la plus proche de cet aliment a été pris comme l'apport correspondant.

Les étapes précédentes ont permis d'obtenir un document qui contient donc les valeurs nutritionnelles de chaque aliment du questionnaire avec leurs variations éventuelles.

1.5. Création du tableau des apports nutritionnels moyens par aliment et par individu L'étape suivante a consisté à multiplier les valeurs moyennes des apports en nutriments par un facteur afin qu'elles correspondent à la portion spécifiée pour chaque aliment dans le questionnaire. Ce facteur est obtenu grâce au document « questionnaire_long_unités.csv » qui contient en particulier la colonne 'Portion' indiquant le nombre de grammes ou de millilitres considérés pour chaque aliment. En effet, les données des bases de données et sources bibliographiques précédentes sont toujours indiquées pour 100g ou 100 mL de l'aliment, il suffit donc de diviser par 100 la valeur dans 'Portion' pour obtenir le facteur multiplicatif (par exemple, pour une Pomme de 150 grammes, le facteur multiplicatif des valeurs nutritionnelles connues pour 100 grammes sera 1,5).

Cela a ainsi permis de créer un document décrivant les apports nutritionnels moyens de chaque aliment du questionnaire dans la portion correcte.

Une fois que l'on possède la valeur nutritionnelle de chaque aliment du questionnaire pour une unique portion, il faut ensuite calculer les valeurs nutritionnelles selon le nombre de portions que l'individu a consommé au cours du dernier mois. On crée ainsi un document qui rassemble, pour toutes les réponses possibles pour chaque aliment du questionnaire, l'apport nutritionnel mensuel correspondant.

À cette étape, on possède d'une part les réponses des utilisateurs volontaires pour répondre au questionnaire alimentaire, et, d'autre part, on possède les apports nutritionnels mensuel pour chaque réponse possible de chaque aliment du questionnaire. Il convient ensuite de calculer la somme de tous les apports pour chaque réponse de l'individu au questionnaire, puis de la diviser par 30 pour obtenir l'apport nutritionnel quotidien moyen estimé pour cet individu.

On obtient ainsi un document décrivant les apports nutritionnels de 70 nutriments chez 288 individus.

1.6. Création du tableau des fréquences de consommation des aliments par individu Les fréquences de consommation de chaque aliment du questionnaire sont obtenues pour chaque individu en prenant le nombre de portions mensuelles de chaque aliment correspondant à la réponse donnée. On divise ensuite ce nombre par 30 pour obtenir ainsi le nombre de portions quotidiennes moyennes consommées pour chaque aliment.

Les quantités de viande (respectivement légumes, fruits ou poissons+fruits de mer) consommées sont alors calculées en additionnant le nombre de portions de viandes (respectivement légumes, fruits ou poissons+fruits de mer) consommées. La quantité Légumes + Fruits correspond à la somme entre les portions précédentes de fruits et celles des légumes. Idem, une division par 30 est réalisée pour obtenir une moyenne estimée journalière.

La diversité des légumes (respectivement fruits, poissons+fruits de mer) est calculée en comptant le nombre de légumes (respectivement fruits, poissons+fruits de mer) différents consommés au moins une fois au cours du dernier mois. Enfin, la diversité "légumes+fruits" est calculée en additionnant les deux scores précédents de diversité des légumes et de diversité des fruits. De même, une division par 30 est réalisée pour obtenir une moyenne estimée journalière.

On obtient ainsi un document décrivant toutes les fréquences de consommation journalières de 145 aliments (+ 9 données de quantité et de diversité) des questionnaires alimentaires de 288 individus.

Deux fichiers ont donc été créés :
- Le fichier « Tableau des apports par ID » décrivant les apports nutritionnels journaliers estimés (70 nutriments) pour 288 individus ayant rempli le questionnaire dans son entièreté.
- Le fichier « Tableau des fréquences par ID» décrivant les fréquences de consommation de 145 aliments du questionnaire alimentaire (+ 9 données de quantité et de diversité) au cours du mois précédent l'analyse pour 288 individus ayant rempli le questionnaire dans son entièreté.

Ces deux fichiers sont ainsi composés au final des données alimentaires de 288 individus. Au début de l'analyse, ont été supprimées les données de 2 individus qui sont des enfants de moins de 4 ans car à cet âge, leur microbiote est encore trop immature pour être représentatif des effets de l'alimentation sur leurs populations bactériennes. On utilise donc les données alimentaires de 286 individus pour les analyses de ce rapport.

### 1.7. Données de microbiote intestinal utilisées

Les résultats ayant servi à l'analyse du microbiote intestinal ont été générés en suivant le processus d'analyse du Dispositif Médical de Diagnostic In Vitro 1test1. En bref, les échantillons fécaux ont été prélevés avec un tube de collecte contenant un conservant afin de permettre le transport à température non controlée depuis l'utilisateur vers le laboratoire d'analyse (DNA/RNA Shield de Zymo Research). L'ADN a ensuite été extrait et le séquençage de la région v3-v4 ARN16s réalisé par le laboratoire IBBL au Luxembourg sur un séquenceur MiSeq d'Illumina selon le protocol en application (peut-être rajouter une référence publication).

Les fichiers de séquençage (fichiers communément appelés fastq) ont ensuite été analysés selon le protocol en application chez Luxia avec le logiciel propriétaire 1TEST1SOFT qui se base sur le logiciel Qiimell et la base de donnée RDP (voir exemple 1).

Au total, 469 individus adultes ont été retenus pour l'analyse.

### 2/ Résultats

Les analyses statistiques ont été réalisées sur les données de la cohorte entière, c'est-à-dire sur le corpuscule total des 469 individus et des 286 données alimentaires sur les nutriments/aliments, ce qui correspond à 215 individus.

Les tests de corrélations de Spearman ont été effectués sur le logiciel R (R Core Team (2019)[20]) par la commande « cor.test(bacteria, food, method = "spearman", exact = FALSE) » pour les aliments et les nutriments. Tandis que les régressions ordinales ont été effectuées avec la fonction clm() du package 'ordinal' après avoir converti les fréquences quotidiennes des aliments consommés par la cohorte considérée en facteurs ordonnés. Ainsi, la fréquence de consommation de ces aliments est considérée comme une variable qualitative ordonnée dont les niveaux correspondent aux réponses choisies par les individus pour chaque aliment, ce qui est donc différent des corrélations de Spearman où cette fréquence de consommation quotidienne des aliments constitue une variable quantitative. Les régressions ordinales sont effectuées sur le logiciel R par la commande « clm(food-bacteria) ».

Sur la cohorte entière (215 individus), on trouve une corrélation entre le beta-carotène et deux des cinq bactéries étudiées, ayant une p-valeur inférieure à 0.05 :

**Tableau 3 : Corrélations >0.1 par ordre de p-value croissante**

| **Bactérie** | **Aliment** | **corrélation** | **P_value** |
|---|---|---|---|
| *Blautia* | Beta-Carotène | 0,14 | 0,04 |
| *Faecalibacterium* | Beta-Carotène | 0,12 | 0,07 |
| *Clostridium_XVIII* | EPA | 0,11 | 0,10 |
| | DHA | | |
| *Clostridium_XVIII* | Beta-Carotène | 0,11 | 0,12 |
| *Clostridium_XVIII* | EPA | 0,10 | 0,16 |
| | DHA | | |

Ce tableau montre la corrélation positive qu'il existe entre le bêta-carotène et les bactéries du genre *Blautia, Faecalibacterium* et *Clostridium XVIII* ainsi qu'entre les oméga-3 (DHA et EPA) et *Clostridium XVIII.*

Plus particulièrement, ces résultats révèlent la corrélation positive significative au seuil de 5% entre l'abondance relative du genre *Blautia* et l'apport en bêta-carotène (p=0.043).

### Exemple 3

Pour valider l'effet bénéfique des 5 nutriments d'intérêt sur le microbiote intestinal, une étude *In Vitro* a été réalisée sur des microbiotes reconstitués à partir de selles d'individus sains et de patients souffrant de la maladie de Parkinson. En particulier, l'effet bénéfique de l'apport de ces nutriments a été évalué à travers l'augmentation de la production de butyrate et d'acides gras à chaine courte en général, ainsi que les changements observés au niveau des métabolites.

Trois donneurs sains et deux donneurs atteints de la maladie de Parkinson ont été recrutés. Pour évaluer les effets de chacun des nutriments sur le microbiote intestinal des donneurs susmentionnés, des simulations coliques à court terme ont été effectuées. L'évaluation de chaque nutriment a été basée 1) sur les effets sur la fermentation microbienne globale (pH) et 2) sur l'activité métabolique microbienne (production de AGCC, de lactate et d'un panel d'environ 400 métabolites).

### Matériel et Méthodes

### Modélisation in vitro du tractus gastro-intestinal

Les approches *in vitro* pour étudier le tractus gastro-intestinal et les processus microbiens intestinaux offrent une excellente configuration expérimentale pour étudier les processus qui se développent dans le microbiome intestinal humain. Le dépistage des effets de composés sélectionnés sur le microbiote intestinal peut être effectué au moyen d'une fermentation fécale réalisée dans un seul réacteur, ce qui permet d'évaluer la capacité de substrats spécifiques à moduler la composition et l'activité microbiennes intestinales. Ces expériences représentent une simulation simplifiée du Simulateur continu de l'écosystème microbien intestinal humain (SHIME^{®}).

### Conservation des échantillons fécaux

Les matières fécales ont été recueillies auprès de cinq donneurs (deux donneurs atteints de la maladie de Parkinson (PD) et trois donneurs sains (HD)).

Les matières fécales ont été recueillies dans des conteneurs anaérobies et conservées à la température du réfrigérateur jusqu'à un traitement ultérieur. Dans les 48 heures suivant la défécation, des suspensions fécales (15 % (p/v)) ont été préparées dans un tampon phosphate et mélangées dans un rapport 1 : 1 avec un cryoprotecteur optimisé en interne dans une chambre anaérobie. Les suspensions obtenues ont été immédiatement surgelées dans de l'azote liquide puis conservées à -80°C (cryostock). Juste avant l'expérience, les échantillons ont été décongelés et immédiatement ajoutés aux réacteurs, de sorte qu'un seul cycle de congélation-décongélation a été appliqué sur chaque inoculum individuel.

### Incubations coliques à court terme

Un test de dépistage à court terme consiste généralement en une incubation colique d'une dose unique d'un composé d'essai dans des conditions représentatives du gros intestin proximal, en utilisant des inoculums bactériens provenant de donneurs sélectionnés comme sources microbiennes. Cinq traitements et une condition de référence (contrôle négatif) ont été testés par donneur. La composition du milieu dans le contrôle et les traitements est exactement la même, mais le contrôle ne contient pas l'ingrédient de test. Le contrôle est inclus pour déterminer les changements de base dans les différents paramètres étudiés (pH, production d'AGCC, lactate et métabolites liés à l'intestin) résultant de la fermentation des nutriments dans le milieu nutritionnel appauvri en glucides qui est introduit avec les ingrédients d'essai dans les réacteurs (voir ci-dessous), pour fournir des nutriments essentiels à la population microbienne. Chaque condition a été testée en une seule répétition chez cinq donneurs.

Un milieu nutritionnel appauvri en glucides (contenant les nutriments basaux du côlon) a été ajouté à chaque réacteur (y compris le contrôle), ainsi que 10 % (v/v) d'un inoculum fécal cryoconservé à 7,5 % (qui a servi de source microbienne) de chaque des cinq donneurs, portant le volume total dans les réacteurs à 70 mL. Des billes porteuses de mucine ont été incluses dans chaque réacteur, pour simuler la couche de mucus du côlon. En plus de cela, six nutriments différents ont été ajoutés (sauf dans le contrôle) dans les concentrations suivantes :
Les poudres de Caroube (galactomannane) et de Konjac (glucomannane) ont été dosés à 4 g/L, ce qui est optimal pour voir les modifications métaboliques résultant de la fermentation des polysaccharides, le cas échéant.

Les beta-carotènes et les oméga-3 ont été dosés à des concentrations reflétant des doses quotidiennes élevées in vivo :
- Caroténoïdes (BC10 et BC30): dose *in vitro* de 50 mg/L, reflétant une dose *in vivo* de 30 mg :
   ▪ Pour 10 % de produit, correspondant à 500 mg/L de produit complet
   ▪ Pour 32,4 % de produit, correspondant à 154 mg/L de produit complet
- Oméga-3 (MEG-3) : dose *in vitro* de 417 mg/L, reflétant une dose *in vivo* de 250 mg :
   ▪ Pour 12,1% de produit, correspondant à 3,4 g/L de produit complet
   • Pour le butyrate, l'équivalent a été dosé de ce qui est normalement obtenu après 48h à court terme expérience, c'est-à-dire 10 mM.

Les réacteurs ont été incubés pendant 48h à 37°C, sous agitation continue (90 rpm) et dans des conditions anaérobies. Les incubations ont été effectuées dans des réacteurs entièrement indépendants avec un volume suffisamment élevé afin non seulement d'assurer une fermentation microbienne robuste, mais également de permettre la collecte de plusieurs échantillons au fil du temps. Le prélèvement d'échantillons permet d'évaluer la production de métabolites et ainsi de comprendre les interactions microbiennes complexes qui se déroulent.

### Critères d'évaluation de l'étude

Activité fermentaire globale par mesure du pH (0h, 24h Et 48h) : le degré d'acidification au cours de l'expérience est une mesure de l'intensité du métabolisme bactérien. Le pH des incubations donne une indication approximative de la vitesse de fermentation des différents produits à tester et sert de contrôle de processus paramètre. Chaque mesure a été effectuée en une seule répétition.

Changements dans la production de métabolites microbiens par mesure des acides gras à chaîne courte (AGCC) (0h, 24h et 48h) : le schéma de production d'AGCC est une évaluation du métabolisme microbien des glucides (acétate, propionate et butyrate) ou du métabolisme des protéines (AGCC ramifié) et peut être comparé aux schémas de fermentation typiques pour le microbiote gastro-intestinal. L'analyse quantitative des AGCC se fait au moyen de gaz capillaire chromatographie couplée à un détecteur à ionisation de flamme (FID). L'isolement des AGCC est réalisé par extraction liquide-liquide. Chaque mesure a été effectuée en une seule répétition.

Changements dans la production de métabolites microbiens par mesure du lactate (0h, 24h Et 48h) : L'intestin humain abrite à la fois des bactéries productrices et utilisant du lactate. Le lactate est produit par les bactéries lactiques et diminue le pH de l'environnement, agissant ainsi également comme agent antimicrobien. L'acide lactique protoné peut pénétrer dans la cellule microbienne, après quoi il se dissocie et libère des protons à l'intérieur de la cellule, entraînant une acidification et la mort des cellules microbiennes. Il peut également être rapidement transformé en propionate et en butyrate par d'autres micro-organismes. La détermination des concentrations de lactate a été réalisée à l'aide du kit EnzytecTM (R-Biopharm). Chaque mesure a été effectuée en une seule répétition.

Changements dans la production de métabolites microbiens par mesure métabolomique (inocula, 0h et 48h) : Les effets sur le métabolome microbien intestinal ont été étudiés en utilisant le profilage métabolomique à large spectre. Pour ce faire, environ 400 métabolites liés à l'intestin ont été ciblés par chromatographie liquide à ultra-haute performance, couplée à la spectrométrie de masse à haute résolution (UHPLC-HRMS).

### Statistiques

Pour évaluer si les effets du traitement en termes de critères d'évaluation étudiés étaient statistiquement pertinents au sein d'un groupe de donneurs donné (patients atteints de la maladie de Parkinson et donneurs sains), des tests T bilatéraux appariés ont été effectués, en tenant compte de tous les donneurs par groupe de donneurs. Chacun des six traitements a été comparé au contrôle à 48h.

### Résultats

### Activité de fermentation

La surveillance du pH au cours d'une incubation colique fournit une bonne indication de la production d'AGCC, de lactate et d'ammonium (NH⁴⁺). En général, une chute de pH est observée initialement en raison de la formation de AGCC/lactate. Cette baisse de pH est souvent suivie d'une augmentation de pH due à la fermentation protéolytique, qui entraîne la production, entre autres, de NH⁴⁺, et en raison de la conversion d'acides plus forts en acides plus faibles par alimentation croisée (par exemple, conversion d'acétate/lactate en propionate/butyrate).

Toutes les incubations se sont déroulées dans des conditions optimales. Dans l'ensemble, le pH variait entre 5,7 et 6,6, ce qui est optimal pour soutenir la croissance des membres de la population microbienne intestinale et pour permettre des interactions d'alimentation croisée. La fermentation était principalement associée à des baisses de pH entre 0 et 24 h, en raison de la production de produits de fermentation acides AGCC et de lactate. Chez les deux patients parkinsoniens, l'oméga-3, la poudre de Konjac et la poudre de Caroube ont eu tendance à entraîner des baisses de pH plus fortes que le contrôle, tandis que le bêta-carotène (10 % et 30 %) et le butyrate ont entraîné des profils de pH similaires à ceux du contrôle. Chez les individus sains, tous les produits sauf le butyrate ont entraîné des baisses de pH significativement plus fortes que le contrôle.

Dans ce cas également, les produits caroténoïdes (10 % et 30 %) et le butyrate ont entraîné les baisses de pH les plus légères. Globalement, le pH est resté relativement stable entre 24-48h.

### Changements dans la production de métabolites microbiens

### Acides gras à chaîne courte (AGCC)

La production d'AGCC résulte du métabolisme des glucides dans le côlon et est liée à divers effets sur la santé. Les AGCC les plus abondamment produits sont l'acétate, le propionate et le butyrate. Alors que l'acétate peut être utilisé comme source d'énergie pour l'hôte et comme substrat potentiel pour la synthèse des lipides dans l'organisme, le propionate réduit la synthèse du cholestérol et des acides gras dans le foie (effet bénéfique sur l'homéostasie métabolique). Le butyrate, quant à lui, est une source d'énergie majeure pour les colonocytes et induit la différenciation de ces cellules (liée à la prévention du cancer). Les effets positifs des substrats étudiés sur la production de AGCC comprennent donc une augmentation de l'acétate, du propionate et/ou du butyrate. Parmi les 5 genres bactériens diminués chez les patients parkinsoniens, 4 sont des producteurs d'AGCC,parmi lesquels 3 (*Roseburia, Faecalibacterium* et *Butyriciccocus*) sont des producteurs de butyrate. La diminution de production de butyrate, résultant de la diminution des bactéries productrices de butyrate, pourrait donc être un mécanisme important dans la prise en charge de ces patients. Ainsi, un complément alimentaire efficace chez de tels patients devra induire en priorité une augmentation de la production de butyrate chez ces patients. L'augmentation de production d'acétate est également intéressante. Le propionate n'étant pas produit par les bactéries diminuées, il n'est pas un des AGCC à cibler.

L'acétate peut être produit par de nombreux microbes intestinaux différents (y compris entre autres *Bifidobacterium spp., Bacteroides spp.* et *Lactobacillus* spp.) et est un métabolite primaire généré par la fermentation de substrats prébiotiques. Les résultats indiquent que chez les deux patients parkinsoniens, les OMEGA-3 (MEG-3), la poudre de konjac et la poudre de caroube ont produit plus d'acétate que le contrôle. Le bêta-carotène BC10 a légèrement stimulé la production d'acétate; le bêta-carotène BC30 et le butyrate (dans le donneur B) ont donné des concentrations d'acétate similaires à celles du contrôle. De même, chez les individus sains, l'OMEGA-3, la poudre de Konjac et la poudre de Caroube ont généré significativement plus d'acétate que le contrôle, et le BC10 avait également tendance à stimuler modérément la production d'acétate. Le BC30 et le butyrate ont donné des concentrations d'acétate similaires à celles du contrôle.

Le propionate peut être produit par différents microbes intestinaux, les producteurs de propionate les plus abondants étant *Bacteroides spp. (phylum Bacteroidetes), Akkermansia muciniphila (phylum Verrucomicrobia)* et *Veillonellaceae (phylum Firmicutes).* Les résultats indiquent que dans les deux cas patients, le Konjac a fortement stimulé la production de propionate et que de légers effets de stimulation ont été observé pour le MEG-3, le butyrate et le galactomannane. Les deux produits caroténoïdes (BC10 et BC30) ont donné des concentrations de propionate similaires à celles du contrôle. De même, chez les individus sains, le Konjac a fortement stimulé la production de propionate, et le MEG-3 et le galactomannane ont eu tendance à stimuler modérément la production de propionate. Les produits caroténoïdes (10 % et 30 %) et le butyrate ont donné des concentrations de propionate similaires à celles du contrôle.

Le butyrate est principalement produit par les membres des familles *Lachnospiraceae et Ruminococcaceae (phylum* = *Firmicutes*) et en particulier *Faecalibacterium, Roseburia* et *Butyriciccocus.* Dans un processus appelé alimentation croisée, ces microbes convertissent l'acétate et/ou le lactate (ainsi que d'autres substrats) en butyrate lié à la santé. Chez les deux patients, le butyrate de sodium a fortement stimulé la production de butyrate (compte tenu du fait que les 10 mM ajoutés au produit n'étaient pas complètement dissous dès le départ, mais se sont dissous au fur et à mesure de l'incubation). Les autres produits ont donné des concentrations de butyrate similaires à celles du contrôle. Au contraire, chez les individus sains, le butyrate de sodium n'a pas stimulé la production de butyrate (en tenant compte également de la dissolution du produit pendant l'incubation), mais dans cette population, le Konjac a généré systématiquement plus de butyrate que le blanc. Les autres produits (BC10, BC30, MEG-3 et galactomannane) n'ont pas modifié significativement la production de butyrate.

### Marqueurs du métabolisme des protéines : AGCC ramifié

Les AGCC moins abondants comprennent les AGCC ramifiés (isobutyrate, isovalérate et isocaproate). La production de AGCC ramifiés résulte de l'activité microbienne protéolytique, qui est associée à la formation de sous-produits toxiques tels que le p-crésol. Par conséquent, une production élevée de AGCC ramifiés dans le côlon a été associée à des effets néfastes sur la santé. En conséquence, les produits qui réduisent la production d'AGCC ramifiés sont considérés comme bénéfiques pour la santé.

Nos résultats montrent que chez les patients parkinsoniens et chez les individus sains, le MEG-3 et le Konjac ont réduit la production de AGCC ramifiés, tandis que les effets des autres produits étaient moins uniformes parmi les donateurs. Cependant, aucun des 5 nutriments testés n'a stimulé la production de AGCC ramifié.

### Production de lactate

L'intestin humain abrite des bactéries productrices et consommatrices de lactate. Le lactate est produit par les bactéries lactiques (bifidobactéries et lactobacilles) et diminue le pH de l'environnement. Surtout à faible pH, le lactate peut exercer de puissants effets antimicrobiens contre les agents pathogènes, car l'acide lactique protoné peut pénétrer dans la cellule microbienne, après quoi il se dissocie et libère des protons à l'intérieur de la cellule, entraînant une acidification et la mort des cellules microbiennes. Un autre effet bénéfique du lactate résulte de sa conversion en butyrate et/ou propionate par des micro-organismes spécifiques. Des effets stimulants sur la production de lactate n'ont pas été observés après l'ajout des 5 nutriments testés, mais peuvent s'être produits au début de l'incubation.

### Production de dopamine

Les présents résultats ont également mis au jour que la mise en contact de Galactomannane permet de stimuler la production de Dopamine chez les patients souffrant de la maladie de Parkinson et chez les personnes saines (cf. figure 6).

Cette observation surprenante renforce l'intérêt d'ajouter du Galactomannane dans un complément alimentaire destiné à être administré à ces patients.

### Conclusion

Les profils de pH ont indiqué que les processus de fermentation dans les simulations coliques se sont déroulés dans des conditions optimales pour soutenir la croissance d'une grande diversité de membres de la population microbienne intestinale, permettant des interactions d'alimentation croisée (conduisant à la production de métabolites secondaires bénéfiques comme le butyrate). Cela a fourni la base de référence idéale pour évaluer les propriétés prébiotiques des produits étudiés.

Le konjac et, dans une moindre mesure, le MEG-3 et le galactomannane ont stimulé la production d'acétate et de propionate dans les deux groupes de donneurs, probablement attribués à une redondance fonctionnelle. Cela a abaissé le pH du milieu d'incubation.

Le butyrate de sodium, un traitement généralement appliqué pour administrer du butyrate chez les patients parkinsoniens, a stimulé la production de butyrate dans la population parkinsonienne, mais pas dans la population en bonne santé.

### REFERENCES BIBLIOGRAPHIQUES

[1] Aho VTE, Pereira PAB, Voutilainen S, Paulin L, Pekkonen E, Auvinen P, et al. Gut microbiota in Parkinson's disease: Temporal stability and relations to disease progression. EBioMedicine 2019; 44: 691-707.
[2] Boertien JM, Pereira PAB, Aho VTE, Scheperjans F. Increasing Comparability and Utility of Gut Microbiome Studies in Parkinson's Disease: A Systematic Review. J Parkinsons Dis 2019; 9: S297-312.
[3] Bolyen E, Rideout JR, Dillon MR, Bokulich NA, Abnet CC, Al-Ghalith GA, et al. Reproducible, interactive, scalable and extensible microbiome data science using QIIME 2. Nat Biotechnol 2019; 37: 852-7.
[4] Braak H, De Vos RAI, Bohl J, Del Tredici K. Gastric α-synuclein immunoreactive inclusions in Meissner's and Auerbach's plexuses in cases staged for Parkinson's disease-related brain pathology. Neurosci Lett 2006; 396: 67-72.
[5] Chaudhuri KR, Healy DG, Schapira AH V, National Institute for Clinical Excellence. Non-motor symptoms of Parkinson's disease: diagnosis and management. Lancet Neurol 2006; 5: 235-45.
[6] Chiricozzi E, Lunghi G, Di Biase E, Fazzari M, Sonnino S, Mauri L. GM1 ganglioside is a key factor in maintaining the mammalian neuronal functions avoiding neurodegeneration. Int J Mol Sci 2020; 21: 1-29.
[7] Cole JR, Wang Q, Fish JA, Chai B, McGarrell DM, Sun Y, et al. Ribosomal Database Project: Data and tools for high throughput rRNA analysis. Nucleic Acids Res 2014; 42: 633-42.
[8] Cryan JF, O'Riordan KJ, Sandhu K, Peterson V, Dinan TG. The gut microbiome in neurological disorders. Lancet Neurol 2020; 19: 179-94.
[9] Dauer W, Przedborski S. Parkinson's disease: mechanisms and models. Neuron 2003; 39: 889-909.
[10] Friedland RP. Mechanisms of Molecular Mimicry Involving the Microbiota in Neurodegeneration. J Alzheimer's Dis 2015; 45: 349-62.
[11] Hill-Burns EM, Debelius JW, Morton JT, Wissemann WT, Lewis MR, Wallen ZD, et al. Parkinson's disease and Parkinson's disease medications have distinct signatures of the gut microbiome. Mov Disord 2017; 32: 739-49.
[12] Kalia L V., Lang AE. Parkinson's disease. Lancet 2015; 386: 896-912
[13] Keshavarzian A, Green SJ, Engen PA, Voigt RM, Naqib A, Forsyth CB, et al. Colonic bacterial composition in Parkinson's disease. Mov Disord 2015; 30: 1351-60.
[14] Kupsky WJ, Grimes MM, Sweeting J, Bertsch R, Cote LJ. Parkinson's disease and megacolon: Concentric hyaline inclusions (lewy bodies) in enteric ganglion cells. Neurology 1987; 37: 1253-5
[15] Nishiwaki H, Ito M, Ishida T, Hamaguchi T, Maeda T, Kashihara K, et al. Meta-Analysis of Gut Dysbiosis in Parkinson's Disease. Mov Disord 2020: mds.28119.
[16] Petrov VA, Saltykova I V., Zhukova IA, Alifirova VM, Zhukova NG, Dorofeeva YB, et al. Analysis of gut microbiota in patients with parkinson's disease. Bull Exp Biol Med 2017; 162: 734-7.
[17] Pfeiffer RF. Non-motor symptoms in Parkinson's disease. Park Relat Disord 2016; 22: S119-22.
[18] Pietrucci D, Cerroni R, Unida V, Farcomeni A, Pierantozzi M, Mercuri NB, et al. Dysbiosis of gut microbiota in a selected population of Parkinson's patients. Park Relat Disord 2019; 65: 124-30.
[19] Raza C, Anjum R, Shakeel N ul A. Parkinson's disease: Mechanisms, translational models and management strategies. Life Sci 2019; 226: 77-90.
[20] R Core Team (2019). R : A language and environment for statistical computing. R Foundation for Statistical Computing, Vienna, Austria. Disponible sur https://www.R-project.org/
[21] Moisan F, Wanneveich M, Kab S, Moutengou É, Boussac-Zarebska M, Carcaillon-Bentata L, et al. Fréquence de la maladie de Parkinson en France en 2015 et évolution jusqu'en 2030. Bull épidémiologique Hebd 2018; 8-9: 128-40.
[22] Alejandra Escobar-Zepeda, Elizabeth Ernestina Godoy-Lozano, Luciana Raggi, Lorenzo Segovia, Enrique Merino, Rosa Maria Gutiérrez-Rios, Katy Juarez, Alexei F. Licea-Navarro, Liliana Pardo-Lopez Et Alejandro Sanchez-Flores. Analysis of sequencing strategies and tools for taxonomic annotation: Defining standards for progressive metagenomics. Scientific Reports volume 8, Article number: 12034 (2018).
[23] Bogacz-Radomska L. and Harasym J.. B-Carotene-properties and production methods. Food Quality and Safety, 2018, 00, 1-6.
[24] Bolyen E et al. Reproducible, interactive, scalable and extensible microbiome data science using QIIME 2. Nat Biotechnol. 2019 Aug;37(8):852-857.
[25] Cole JR, Wang Q, Fish JA, Chai B, McGarrell DM, Sun Y, Brown CT, Porras-Alfaro A, Kuske CR, Tiedje JM.. Ribosomal Database Project: data and tools for high throughput rRNA analysis. Ribosomal Database Project: data and tools for high throughput rRNA analysis. Nucleic Acids Res. 2014 Jan;42.
[26] Benjamini Y, Hochberg Y (1995). "Controlling the false discovery rate: a practical and powerful approach to multiple testing" (PDF). Journal of the Royal Statistical Society, Series B. 57 (1): 289-300
[27] Heintz-Buschart A, Pandey U, Wicke T, Sixel-Döring F, Janzen A, Sittig-Wiegand E, et al. The nasal and gut microbiome in Parkinson's disease and idiopathic rapid eye movement sleep behavior disorder. Mov Disord 2018; 33: 88-98.
[28] Hopfner F, Künstner A, Müller SH, Künzel S, Zeuner KE, Margraf NG, et al. Gut microbiota in Parkinson disease in a northern German cohort. Brain Res 2017; 1667: 41-5.
[29] Jane G Muir, Susan J Shepherd, Ourania Rosella, Rosemary Rose, Jacqueline S Barrett, Peter R Gibson. Fructan and free fructose content of common Australian vegetables and fruit. J Agric Food Chem. 2007 Aug 8;55(16):6619-27.
[30] Jane G. Muir, Rosmary Rose, Ourania Rosella, Kelly Liels, Jacqueline S. Barrett, Susan J. Shepherd, and Peter R. GibsonMeasurement of Short-Chain Carbohydrates in Common Australian Vegetables and Fruits by High-Performance Liquid Chromatography (HPLC). J. Agric. Food Chem. 2009, 57, 2, 554-565.
[31] La Rosa SL, Leth ML, Michalak L, Hansen ME, Pudlo NA, Glowacki R, Pereira G, Workman CT, Arntzen MØ, Pope PB, Martens EC, Hachem MA, Westereng B. The human gut Firmicute Roseburia intestinalis is a primary degrader of dietary B-mannans. Nat Commun. 2019 Feb 22;10(1):905
[32] Taghizadeh M, Tamtaji OR, Dadgostar E, Daneshvar Kakhaki R, Bahmani F, Abolhassani J, Aarabi MH, Kouchaki E, Memarzadeh MR, Asemi Z. The effects of omega-3 fatty acids and vitamin E co-supplementation on clinical and metabolic status in patients with Parkinson's disease: A randomized, double-blind, placebo-controlled trial. Neurochem Int. 2017 Sep;108:183-189.
[33] Cantu-Jungles TM, Rasmussen HE, Hamaker BR. Potential of Prebiotic Butyrogenic Fibers in Parkinson's Disease. Front Neurol. 2019 Jun 20;10:663

## Revendications

1. Formulation orale contenant une quantité efficace de bêta-carotène, de glucomannane et de galactomannane.

2. Formulation orale selon la revendication 1, **caractérisée en ce qu'**elle contient en outre une quantité efficace de butyrate.

3. Formulation orale selon la revendication 1 ou 2, **caractérisée en ce qu'**elle contient en outre une quantité efficace d'omégas-3.

4. Formulation orale selon l'une des revendications 1 - 3, **caractérisée en ce qu'**elle est conditionnée sous forme de gélule, de cachet ou de poudre.

5. Formulation orale selon la revendication 1, contenant une quantité efficace de poudre de carotte.

6. Formulation orale selon la revendication 1, contenant une quantité efficace de poudre de konjac.

7. Formulation orale selon la revendication 1, contenant une quantité efficace de poudre de caroube.

8. Formulation orale selon la revendication 1, comprenant de la poudre de carotte, de la poudre de konjac, et de la poudre de caroube.

9. Procédé pour augmenter l'abondance relative des genres bactériens *Roseburia, Blautia, Faecalibacterium, Butyricicoccus* et/ou *Clostridium_XVIII* au sein du microbiote intestinal d'un individu, **caractérisé en ce qu'**il comprend l'administration de la formulation selon l'une des revendications 1 à 8.

10. Procédé selon la revendication 9, **caractérisé en ce que** ledit individu présente un microbiote pauvre en bactéries du genre *Roseburia, Blautia, Faecalibacterium, Butyricicoccus* et/ou *Clostridium_XVIII.*

11. Procédé selon la revendication 9, **caractérisé en ce que** ledit individu est atteint d'une maladie neurodégénérative telle que la maladie de Parkinson.

12. Procédé pour améliorer la qualité de vie des patients atteints de la maladie de Parkinson, comprenant l'administration à ces patients de la formulation selon l'une quelconque des revendications 1 à 8.

13. Utilisation du bêta-carotène pour améliorer la qualité de vie de patients atteints de la maladie de Parkinson.

14. Utilisation du galactomannane pour améliorer la qualité de vie de patients atteints de la maladie de Parkinson.

15. Utilisation du glucomannane pour améliorer la qualité de vie de patients atteints de la maladie de Parkinson.
